# EUROPEAN PATENT SPECIFICATION

(11) **EP 1 372 462 B1**
(45) Date of publication and mention of the grant of the patent: **24.08.2005**
(21) Application number: 02718705.3
(22) Date of filing: 02.04.2002
(51) Int. Cl.: A61B 3/032

(54) **METHOD AND DEVICE FOR ASSESSING VISUAL ACUITY**
VERFAHREN UND VORRICHTUNG ZUR ERFASSUNG DER SEHSCHÄRFE
PROCEDE ET DISPOSITIF DE CONTROLE DE L'ACUITE VISUELLE

(30) Priority: 30.03.2001 NL 1017741
(43) Date of publication of application: 02.01.2004
(73) Proprietor: Laufer, Manfred, 6941 Niederanven (LU)
(72) Inventor: Laufer, Manfred, 6941 Niederanven (LU)
(74) Representative: van Westenbrugge, Andries
(86) International application number: PCT/NL2002/000214
(87) International publication number: WO 2002/078529

(56) References cited:
- EP-A- 0 261 963
- EP-A- 0 911 792
- EP-A- 0 925 012
- WO-A-98/18381
- US-A- 5 515 118
- US-A- 5 530 492

## Description

The present invention relates to a method and device for assessing the visual acuity of a person.

European Patent Application EP-A 0 925 012 discloses a method and device for testing the visual acuity of a person by displaying symbols on a screen. Symbols, such as letters, numerals and the like, are displayed on the screen, both the background and the symbols being displayed on a screen with a fixed resolution (pixel size). Assessment of the visual acuity (near and far) takes place by means of an adjustable optical correction, through which the symbols on the screen are viewed. The symbols can be displayed in various sizes.

European Patent Application EP-A 1 042 984 discloses a device for the presentation of an optometric card. The device is provided with separate test windows for the left and the right eye. An optotype, consisting of a rotary optometric disc, is positioned optically a certain distance away from the windows. The test is conducted automatically, supported by verbal messages.

The known optometers make use of known presentations of test objects, frequently in the form of printed cards with symbols, such as the Monnoyer card (distance test) or Parinaud card (test at reading distance). The accuracy of the known tests is sometimes insufficient, for example if a person is frequently tested using the same card symbols and, as it were, instead of the visual acuity, the person's memory is tested.

The aim of the present invention is to provide an improved method and device for the assessment of visual acuity, in particular for the prescription of spectacle lenses or contact lenses.

The present invention therefore provides, in a first aspect, a method for the assessment of visual acuity of a person, comprising the steps of displaying, at a preset distance, at least one shape on a background, the background being displayed with a first pixel size and the at least one shape with a second pixel size, the second pixel size being greater than the first pixel size, adjustment of a correction device for optical correction of the visual acuity until the at least one shape is distinguishable from the background, and determining the correction for visual acuity on the basis of the setting of the correction device.

Surprisingly it has been found that the visual acuity of a person can also be assessed by making use of a symbol or shape on a background, where the background has a finer pixel size than the shape. Because of the small pixel size, the background creates the impression of an even surface, against which the shape with the larger pixels stands out. Using this method the visual acuity can be determined more rapidly and more accurately. Subjective factors of recognition and the like play a lesser role, as a result of which a more objective assessment of the visual acuity becomes possible. The at least one shape can be a numeral, letter or a specific shape.

In one embodiment of the present invention the relative pixel density of the background and the at least one shape is essentially the same. The pixel density is defined as the number of pixels per unit surface area multiplied by the surface area of one pixel. Someone who is a long distance away or has poorly corrected eyes will in this case see both the background and the at least one shape as an even, homogeneous surface. Someone with good or properly corrected visual acuity will, however, detect a grainy shape against an even background.

Preferably the background and the at least one shape are displayed using a combination of at least two colours of pixels. Black and white pixels will, for example, yield a grey background, yellow and blue pixels a green background and blue and black pixels a dark blue background. If the visual acuity is properly corrected the at least one shape will stand out against the background, as a grainy grey shape against the grey background, yellow and blue dots against a green background and blue pixels against a dark blue background.

In a further embodiment of the present invention the background is displayed in a first colour and the at least one shape in at least a second colour. The background can, for example, consist of white pixels and the shape of black pixels, or vice versa. A further colour (green, blue, red, ... ) can also be used for the shape, as a result of which the contrast can be improved.

In order to increase the accuracy of the assessment of visual acuity, adjustment of the correction device is preferably carried out a number of times and the corrections for visual acuity determined from the average of the respective settings of the correction device. A more accurate and objective assessment of the visual acuity becomes possible by this means.

In a further embodiment the method is carried out in an alternating manner, the at least one shape being displayed in a region of visible light with a short wavelength and in a region of visible light with a long wavelength. The refraction of the eye is dependent on the wavelength. In order to arrive at a better assessment of the visual acuity, two measurements are carried out at the ends of the visible spectrum and, for example, the average is taken as the result.

The at least one shape can be displayed in a number of variants. For instance, the at least one shape can be displayed alternately with the first and the second pixel size, for example with a frequency of less than 10 Hertz. The flashes of the shape with the second pixel size are discernible only to a person with good (corrected) visual acuity. It is also possible to make the at least one shape turn in the display or to make the at least one shape move (back and forth, up and down) in the display. All these variants increase the unpredictability of the measurement and thus give a more objective assessment of the visual acuity.

In order to be able to determine the extent of astigmatism, in a further embodiment the at least one shape comprises parallel lines that are displayed such that they can be turned between 0 and 180 degrees. The shape is then most clearly visible when the lines run parallel to the axis of the astigmatism of the eye. Preferably, the person to be tested can change the orientation of the parallel lines in the shape him- or herself, for example with the aid of a joystick.

In order to make quantitative measurement of the visual acuity possible, in yet a further embodiment the second pixel size is variable.

In a further aspect the present invention relates to a device for the assessment of visual acuity of a person, comprising a correction device with an adjustable optical correction for visual acuity, a display screen that is placed a predetermined distance away from the correction device, and control means that are connected to the display screen and the correction device, the control means being equipped to control the display screen and to control the correction device according to the present method. Using such a device it is possible for a person to assess his or her visual acuity him- or herself.

In one embodiment the device further comprises an optical system that is equipped for projection of the display screen at reading distance from the correction device. By this means it is possible to assess the visual acuity with regard to near distance (reading distance) as well.

In yet a further aspect the present invention relates to an optometric card comprising a background with a first pixel size and at least one shape with a second pixel size, the second pixel size being larger than the first pixel size. This card can be used in existing devices for the assessment of visual acuity, as a replacement for the existing optometric cards.

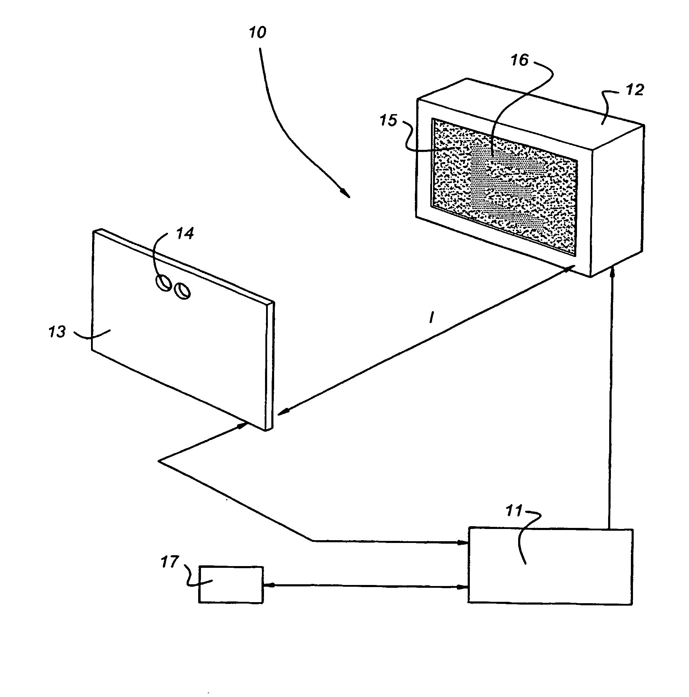

The present invention is described in more detail on the basis of an illustrative embodiment of the present invention, with reference to the appended drawing, in which
Fig. 1 shows a simplified representation of a set-up of the device according to the present invention.

A simple representation of a possible set-up of a device 10 according to the present invention is shown in Fig. 1. A display screen 12 is arranged a predetermined distance 1, for example four metres, away from a correction device 13. A user of the device is able to look through an opening 14 in the correction device 13 towards the display screen 12. Optical correction lenses, the strength of which is adjustable (by, for example, adding lenses) can be placed in the opening 14. The opening 14 is made for two eyes, the two halves of the opening 14 being adjustable independently of one another.

The display screen 12 and the correction device 13 are connected to a control device 11. The control device 11 controls the setting of the correction device 13 as a function of input signals which, for example, are received via an adjuster element 17, such as a joystick, that is connected to the control device 11. The control device 11 also controls the display screen 12. The control device 11 can be provided with memory means in some form or other (such as RAM memory, a DVD, video tape, etc.). These can be used, on the one hand, for recording results obtained and, on the other hand, for driving the control device 11 on the basis of a stored program.

For checking or determining the visual acuity of a person the device 10 is preferably set up in a somewhat darkened room. The control device 11 controls the display screen 12 in such a way that a shape 16 (or multiple shapes) is displayed on a background 15. The background 15 is displayed with a specific pixel size, as a result of which, some distance away, this gives the impression of an even surface. The shape 16 is displayed with (a smaller number of) larger pixels. The pixel size of the shape 16 is thus larger than the pixel size of the background 15.

Preferably the relative pixel densities of the background and of the at least one shape are essentially the same. The pixel density is defined as the number of pixels per unit surface area multiplied by the surface area of one pixel. By this means someone with poor (corrected) visual acuity will see an even homogeneous surface and someone with properly corrected visual acuity will see a grainy shape 16 on an even background 15. The background 15 and shape 16 can be displayed using a combination of at least two colours of pixels, for example black/white, yellow/blue, red/black or blue/black. If correction is correct a grainy shape 16 will then stand out against the even background 15, a shape 16 consisting of yellow and blue dots against a green background, or red or blue pixels against a dark red or dark blue background 15.

The shape 16 can be a numeral or letter, or the outlines of an everyday article. Preferably, the shape 16 is of a size such that a large part of the background 15 is covered. The control device 11 controls the display screen 12 in such a way that the shape 16 is preferably varied so that this is unpredictable for the person.

The shape 16 is visible only if the visual acuity of the person is good or has been well corrected, so that the resolving power of the eyes is able to differentiate the coarser pixels of the shape 16.

The person is able to vary the strength of the correction lenses in the correction device 13 with the aid of the adjuster element 17 and is also able to indicate when the shape 16 is most clearly visible to him or her. Optionally, the person can indicate outer limits, that is to say when the shape 16 becomes visible and when it disappears. The control device 11 can then, for example, calculate a mid position which indicates the proper correction for the person.

The shape 16 is preferably displayed in a different colour from the background 15 (for example white shape 16 on black background 15, or vice versa). The shape 16 can also be displayed in more than one colour, as a result of which it stands out better against the background 15.

In an alternative embodiment two measurements are carried out by the device 10 in question. For a first measurement the shape 16 is displayed in a colour of visible light with a short wavelength, for example violet-blue. For a subsequent measurement the shape 16 is displayed in a colour at the other end of the visible spectrum, for example in red. The refraction of the lens of the eye differs for different wavelengths and by means of the two measurements in violet-blue and in red the ideal correction value can be calculated (for example as an average).

In order to increase the accuracy even further, the shape 16 can be displayed alternately with the fine pixels and with the coarse pixels, with a frequency of, for example, less than 10 times per second. The shape 16 displayed as flashes is visible only if the visual acuity has been well corrected.

The control device 11 can also be equipped to make the shape turn in the display, the shape 16 being, for example, a rotating cross, a rotating wheel with spokes, or rotating windmill sails. It is also possible to display the shape 16 as a moving shape 16. This ensures that the display of shape 16 is even more unpredictable, as a result of which the measurement with the device 10 in question can be carried out more objectively.

In a special embodiment of the present invention the degree of astigmatism can also be determined. In this case the shape 16 is displayed on the display screen 12 as a shape with thin parallel lines. The angle of the parallel lines can be adjusted, for example by means of the abovementioned adjuster element 17. The parallel lines of the shape 16 are clearest to the person when these run parallel to the axis of the astigmatism. With the aid of the adjuster element 17 the person is able to indicate when the shape 16 is clearest, after which the control means 11 determine the best correction.

In yet a further embodiment the visual acuity of a person can be measured quantitatively by varying the pixel size of the shape 16. The visual acuity is determined by determining the smallest pixel size at which the person still sees a distinction between the shape 16 and the background 15.

The present invention can also be used to determine the visual acuity of a person for prescribing reading glasses. For this purpose the display screen 12 is projected, for example by means of an optical system (with the use of lenses and/or mirrors, not shown) onto a matt glass screen that is located at reading distance from the opening 14 in the correction device 13. The image is reduced to such an extent that the angle of vision for the person remains the same.

In order to make operation of the device 10 as simple and as objective as possible, spoken instructions can be made audible. The instructions contain indications of what the user must look out for and how the device 10 must be operated. It is also possible to ask which numeral, letter or shape is detected. The user can input this into the device 10 with the aid of a speech recognition module or another input device. The input can then be recorded.

## Claims

1. Method for the assessment of visual acuity of a person, comprising the steps of:
- displaying at a preset distance at least one shape (16) on a background (15),
- adjustment of a correction device (13, 14) for optical correction of the visual acuity until the at least one shape (16) is distinguishable from the background (15), and
- determining the correction for visual acuity on the basis of the setting of the correction device (13, 14)
**characterized in that**,
- the background (15) is displayed with a first pixel size and the at least one shape (16) with a second pixel size, the second pixel size being greater than the first pixel size.

2. Method according to Claim 1, wherein the relative pixel density of the background (15) and the at least one shape (16) is essentially the same and the pixel density is defined as the number of pixels per unit surface area multiplied by the surface area of one pixel.

3. Method according to Claim 1 or 2, wherein the background (15) and the at least one shape (16) are displayed using a combination of at least two colours of pixels.

4. Method according to Claim 1 or 2, wherein the background (15) is displayed in a first colour and the at least one shape (16) in at least a second colour.

5. Method according to one of the preceding claims, wherein adjustment of the correction device (13, 14) is carried out a number of times and the corrections for visual acuity determined from the average of the respective settings of the correction device (13, 14).

6. Method according to one of the preceding claims, wherein the method is carried out in an alternating manner, the at least one shape (16) being displayed in a region of visible light with a short wavelength and in a region of visible light with a long wavelength.

7. Method according to one of the preceding claims, wherein the at least one shape (16) is displayed alternately with the first and the second pixel size.

8. Method according to one of the preceding claims, wherein the at least one shape (16) is made to turn in the display.

9. Method according to one of the preceding claims, wherein the at least one shape (16) is made to move in the display.

10. Method according to one of the preceding claims, wherein the at least one shape (16) comprises parallel lines that are displayed such that they can be turned between 0 and 180 degrees.

11. Method according to one of the preceding claims, wherein the second pixel size is variable.

12. Device for the assessment of visual acuity of a person, comprising
a correction device (13, 14) with an adjustable optical correction for visual acuity,
a display screen (12) that is placed a predetermined distance away from the correction device (13, 14),
and control means (11) that are connected to the display screen (12) and the correction device (13, 14), the control means (11) being equipped to control the display screen (12) and to control the correction device (13, 14),
where the control means (11) are arranged to display at least one shape (16) on a background (15),
where the control means (11) are arranged to adjust the optical correction for optical acuity of the correction device (13, 14) until the at least one shape (16) is distinguishable from the background (15) and
where the control means (11) is adapted to determine the correction for visual acuity on the basis of the setting of the correction device (13, 14)
**characterized in that**
the background (15) has a first pixel size and the at least one shape (16) has a second pixel size on the display screen (12), the second pixel size being greater than the first pixel size.

13. Device according to Claim 12, further comprising an optical system that is equipped for projection of the display screen (12) at reading distance from the correction device (13, 14).

## Patentansprüche

1. Verfahren zur Erfassung der Sehschärfe einer Person, das die folgenden Schritte aufweist:
Anzeigen wenigstens einer Form (16) auf einem Hintergrund (15) in einer vorbestimmten Distanz,
Einstellen einer Korrekturvorrichtung (13, 14) zur Korrektur der Sehschärfe bis die wenigstens eine Form (16) von dem Hintergrund (15) unterscheidbar ist, und
Bestimmung der Sehschärfe-Korrektur aufgrund der Einstellung der Korrekturvorrichtung (13, 14),
**dadurch gekennzeichnet,**
**daß** der Hintergrund (15) in einer ersten Pixelgröße und die wenigstens eine Form (16) in einer zweiten Pixelgröße dargestellt wird, wobei die zweite Pixelgröße größer ist als die erste Pixelgröße.

2. Verfahren nach Anspruch 1, wobei die relativen Pixeldichten des Hintergrundes (15) und der wenigstens einen Form (16) im wesentlichen gleich sind und die Pixeldichte definiert ist als Anzahl von Pixeln pro Flächeneinheit multipliziert mit der Fläche eines Pixels.

3. Verfahren nach Anspruch 1 oder 2, wobei der Hintergrund (15) und die wenigstens eine Form (16) durch eine Kombination von wenigstens zwei Pixelfarben angezeigt werden.

4. Verfahren nach Anspruch 1 oder 2, wobei der Hintergrund (15) in einer ersten Farbe und die wenigstens eine Form (16) in wenigstens einer zweiten Farbe angezeigt werden.

5. Verfahren gemäß einem der voranstehenden Ansprüche, wobei das Einstellen der Korrekturvorrichtung (13, 14) einige Male ausgeführt wird und die Sehschärfe-Korrekturen aus dem Mittelwert der entsprechenden Einstellungen der Korrekturvorrichtung (13, 14) bestimmt werden.

6. Verfahren nach einem der voranstehenden Ansprüche, wobei das Verfahren in alternierender Weise derart durchgeführt wird, daß die wenigstens eine Form (16) in einem Bereich des sichtbaren Lichts mit einer kurzen Wellenlänge und in einem Bereich des sichtbaren Lichts mit einer langen Wellenlänge angezeigt wird.

7. Verfahren nach einem der voranstehenden Ansprüche, wobei die wenigstens eine Form (16) abwechselnd mit der ersten und der zweiten Pixelgröße dargestellt wird.

8. Verfahren nach einem der voranstehenden Ansprüche, wobei die wenigstens eine Form (16) dazu vorgesehen ist, sich in der Anzeige zu drehen.

9. Verfahren nach einem der voranstehenden Ansprüche, wobei die wenigstens eine Form (16) dazu vorgesehen ist, sich in der Anzeige zu bewegen.

10. Verfahren nach einem der voranstehenden Ansprüche, wobei die wenigstens eine Form (16) parallele Linien umfaßt, die derart dargestellt werden, daß sie zwischen 0 und 180° gedreht werden können.

11. Verfahren nach einem der voranstehenden Ansprüche, wobei die zweite Pixelgröße veränderlich ist.

12. Vorrichtung zur Erfassung der Sehschärfe einer Person, wobei die Vorrichtung aufweist:
eine Korrekturvorrichtung (13, 14) mit einer einstellbaren Sehschärfe-Korrektur,
einen Bildschirm (12), der in einer vorbestimmten Distanz beabstandet von der Korrekturvorrichtung (13, 14) angeordnet ist und
Steuerungsmittel (11), die mit dem Bildschirm (12) und der Korrekturvorrichtung (13, 14) verbunden sind, wobei die Steuerungsmittel (11) zur Steuerung des Bildschirms (12) und der Korrekturvorrichtung (13, 14) ausgestaltet sind,
wobei die Steuerungsmittel (11) zur Darstellung wenigstens einer Form (16) auf einem Hintergrund (15) ausgestaltet sind,
wobei die Steuerungsmittel (11) zur Einstellung der optischen Sehschärfe-Korrektur der Korrekturvorrichtung (13, 14) eingerichtet sind, bis die wenigstens eine Form (16) vom Hintergrund (15) unterscheidbar ist und
wobei das Steuerungsmittel (11) dazu angepaßt ist, die Sehschärfe-Korrektur auf Basis der Einstellung der Korrekturvorrichtung (13, 14) zu bestimmen,
**dadurch gekennzeichnet,**
**daß** auf dem Bildschirm (12) der Hintergrund (15) eine erste Pixelgröße und die wenigstens eine Form (16) eine zweite Pixelgröße aufweist, wobei die zweite Pixelgröße größer als die erste Pixelgröße ist.

13. Vorrichtung gemäß Anspruch 12, wobei die Vorrichtung darüber hinaus ein optisches System umfaßt, das zur Projektion des Bildschirms (12) auf Leseabstand von der Korrekturvorrichtung (13, 14) ausgestaltet ist.

## Revendications

1. Procédé pour l'évaluation de l'acuité visuelle d'une personne, comprenant les étapes :
- d'affichage à une distance préétablie d'au moins une forme (16) sur un arrière-plan (15),
- de réglage d'un dispositif de correction (13, 14) pour la correction optique de l'acuité visuelle jusqu'à ce que la forme (16) soit distinguable de l'arrière-plan (15), et
- de détermination de la correction pour l'acuité visuelle sur la base du réglage du dispositif de correction (13, 14),
**caractérisé en ce que**,
- l'arrière-plan (15) est affiché avec une première taille de pixel et la forme (16) avec une seconde taille de pixel, la seconde taille de pixel étant supérieure à la première taille de pixel.

2. Procédé selon la revendication 1, dans lequel la densité de pixels relative de l'arrière-plan (15) et de la forme (16) est essentiellement la même et la densité de pixels est définie comme le nombre de pixels par unité de surface multiplié par l'aire d'un pixel.

3. Procédé selon la revendication 1 ou 2, dans lequel l'arrière-plan (15) et la forme (16) sont affichés en utilisant une combinaison d'au moins deux couleurs de pixels.

4. Procédé selon la revendication 1 ou 2, dans lequel l'arrière-plan (15) est affiché dans une première couleur et la forme (16) dans au moins une seconde couleur.

5. Procédé selon l'une des revendications précédentes, dans lequel le réglage du dispositif de correction (13, 14) est effectué un certain nombre de fois et les corrections pour l'acuité visuelle sont déterminées à partir de la moyenne des réglages respectifs du dispositif de correction (13, 14).

6. Procédé selon l'une des revendications précédentes, dans lequel le procédé est effectué de manière alternée, la forme (16) étant affichée dans une région de lumière visible avec une petite longueur d'onde et dans une région de lumière visible avec une grande longueur d'onde.

7. Procédé selon l'une des revendications précédentes, dans lequel la forme (16) est affichée alternativement avec la première et la seconde taille de pixel.

8. Procédé selon l'une des revendications précédentes, dans lequel on fait tourner la forme (16) dans l'affichage.

9. Procédé selon l'une des revendications précédentes, dans lequel on déplace la forme (16) dans l'affichage.

10. Procédé selon l'une des revendications précédentes, dans lequel la forme (16) comporte des lignes parallèles qui sont affichées de telle sorte qu'on peut les faire tourner entre 0 et 180 degrés.

11. Procédé selon l'une des revendications précédentes, dans lequel la seconde taille de pixel est variable.

12. Dispositif pour l'évaluation de l'acuité visuelle d'une personne, comprenant
un dispositif de correction (13, 14) avec une correction optique réglable pour l'acuité visuelle,
un écran d'affichage (12) qui est placé à une distance prédéterminée du dispositif de correction (13, 14),
et des moyens de contrôle (11) qui sont connectés à l'écran d'affichage (12) et au dispositif de correction (13, 14), les moyens de contrôle (11) étant équipés pour contrôler l'écran d'affichage (12) et pour contrôler le dispositif de correction (13, 14),
où les moyens de contrôle (11) sont disposés pour afficher au moins une forme (16) sur un arrière-plan (15),
où les moyens de contrôle (11) sont disposés pour régler la correction optique pour l'acuité optique du dispositif de correction (13, 14) jusqu'à ce que la forme (16) soit distinguable de l'arrière-plan (15) et
où les moyens de contrôle (11) sont adaptés pour déterminer la correction pour l'acuité visuelle sur la base du réglage du dispositif de correction (13, 14)
**caractérisé en ce que**
l'arrière-plan (15) présente une première taille de pixel et la forme (16) présente une seconde taille de pixel sur l'écran d'affichage (12), la seconde taille de pixel étant supérieure à la première taille de pixel.

13. Dispositif selon la revendication 12, comprenant en outre un système optique qui est équipé pour la projection de l'écran d'affichage (12) à une distance de lecture du dispositif de correction (13, 14).
